# EUROPÄISCHE PATENTANMELDUNG

(11) **EP 4 553 036 A1**
(43) Veröffentlichungstag der Anmeldung: **14.05.2025**
(21) Anmeldenummer: 24211387.6
(22) Anmeldetag: 07.11.2024
(51) Int. Cl.: B67C 7/00

(54) **VERFAHREN UND ANLAGE ZUM BEHANDELN VON DOSEN**

(30) Priorität: 09.11.2023 DE 102023131037
(71) Anmelder: KRONES AG, 93073 Neutraubling (DE)
(72) Erfinder: NEUBAUER, Michael, 93073 Neutraubling (DE); WINTER, Ute, 93073 Neutraubling (DE)
(74) Vertreter: v. Bezold & Partner Patentanwälte - PartG mbB

(57) **Zusammenfassung**

Die Erfindung betrifft u.a. ein Verfahren zum Behandeln von Dosen (12) in einer Dosenbehandlungsanlage (10). Das Verfahren weist ein Zuführen eines Sterilisationsmediums in eine Dose (12) mittels einer Sterilisationsvorrichtung (14) und ein Innensterilisieren der Dose (12) durch das zugeführte Sterilisationsmedium, während die Dose (12) zu einer Füllstation (24) transportiert wird, auf. Das Verfahren weist ferner ein Spülen der Dose (12) in der Füllstation (24) mit einem Spülgas, wodurch das Sterilisationsmedium aus der Dose (12) ausgespült wird, und ein Füllen der gespülten Dose (12) in der Füllstation (24) mit einem Füllgut auf.

## Beschreibung

### Technisches Gebiet

Die Erfindung betrifft ein Verfahren zum Behandeln von Dosen mit einem Innensterilisieren der Dosen. Die Erfindung betrifft ferner eine Dosenbehandlungsanlage mit einer Sterilisationsvorrichtung.

### Technischer Hintergrund

In Dosenabfüllanlagen können gefüllte und verschlossene Getränkedosen in einem Pasteur thermisch behandelt werden. Dies sichert die mikrobiologische Sicherheit des Füllgutes und schützt somit den Verbraucher und verlängert die Haltbarkeit des Füllgutes.

Da durch die Pasteurisation alle potenziell vorhandenen mikrobiologischen Schädlinge abgetötet werden, wird herkömmlich kein besonderer hygienischer Aufwand betrieben, um mikrobiologische Verkeimung, die das Füllgut innerhalb der Mindesthaltbarkeit verderben könnte, vor dem Pasteurisieren zu vermeiden.

Nachteilig an der Pasteurisation kann sein, dass diese zwar sehr sicher, aber energetisch sehr aufwendig ist. Zudem kann das Füllgut beim Pasteurisieren in der Dose thermisch stark belastet werden. Dies kann negative Auswirkungen auf den Geschmack des Füllguts haben. Auch können wertvolle Inhaltsstoffe wie Vitamine im thermischen Pasteurisationsprozess geschädigt werden.

Der Erfindung liegt die Aufgabe zu Grunde, eine verbesserte Technik zum Behandeln von Dosen zu schaffen, die vorzugsweise schneller, wirksamer und/oder effizienter als die herkömmliche Technik, z. B. mit Pasteurisiervorrichtung, ist.

### Zusammenfassung der Erfindung

Die Aufgabe wird gelöst durch die Merkmale der unabhängigen Ansprüche. Vorteilhafte Weiterbildungen sind in den abhängigen Ansprüchen und der Beschreibung angegeben.

Ein Aspekt der vorliegenden Offenbarung betrifft ein Verfahren zum Behandeln von Dosen (z. B. Getränkedosen oder Konservendosen) in einer Dosenbehandlungsanlage. Das Verfahren weist ein Zuführen (z. B. Einblasen oder Eindüsen) eines Sterilisationsmediums, vorzugsweise aufweisend Wasserstoffperoxid, in eine Dose mittels einer Sterilisationsvorrichtung auf. Das Verfahren weist ein Innensterilisieren der Dose durch das zugeführte Sterilisationsmedium, während die Dose zu einer Füllstation (z. B. einer Dosenfüllvorrichtung, z. B. eines Dosenfüllerkarussells) transportiert wird (und vorzugsweise die Füllstation erreicht), auf. Das Verfahren weist ein Spülen der Dose in der Füllstation mit einem Spülgas, vorzugsweise Kohlenstoffdioxid, wodurch das Sterilisationsmedium aus der Dose (z. B. vollständig) ausgespült wird, auf. Das Verfahren weist ein Füllen der gespülten Dose in der Füllstation mit einem (z. B. flüssigen oder pastösen) Füllgut auf.

Vorteilhaft ermöglicht das Verfahren nicht einfach nur eine Sterilisation der Dosen vor dem Füllen. Zusätzlich ermöglicht das Verfahren vorteilhaft, dass die Schritte des Entfernens (Ausblasens) des Sterilisationsmediums aus den Dosen und des Spülens der Dosen vor dem Füllen integriert bzw. gemeinsam ausgeführt werden. In anderen Worten, die vor dem Füllen einer Dose durchgeführte Spülung, z. B. mit CO2, zum Verringern eines Sauerstoffgehalts in der Dose wird (auch) dazu genutzt, das Sterilisationsmedium aus der Dose zu spülen, wodurch ein separater Schritt zum Entfernen des Sterilisationsmediums noch in der Sterilisationsvorrichtung entfallen kann. D.h., beim Spülen kann nunmehr sowohl Luft mit dem darin enthaltenen Sauerstoff als auch das Sterilisationsmedium aus der Dose gespült werden. Durch das Zusammenführen dieser Schritte wird der Gesamtprozess vorteilhaft schneller, wirksamer und effizienter, wie nachfolgend beispielhaft beschrieben ist.

Das Verfahren kann vorteilhaft besonders schnell sein, da eine gesonderte, mehrsekündige Ausblaszeit vor der Dosenfüllvorrichtung bzw. vor der Füllstation entfallen kann. Je nach Maschinenleistung können einige Meter Transportstrecke eingespart werden. Die Transportzeit zur Dosenfüllvorrichtung und die Prozesszeit in der Füllstation bis zum Spülen sind zusätzliche Einwirkzeiten für das Sterilisationsmedium. Somit kann vorteilhaft die Einwirkzeit und damit die Transportzeit in der Sterilisationsvorrichtung ebenfalls verkürzt werden, wodurch die Sterilisationsvorrichtung vergleichsweise klein ausgeführt werden kann.

Das Verfahren kann vorteilhaft besonders wirksam sein, da eine vergleichsweise lange Einwirkzeit zum Innensterilisieren zur Verfügung steht. Das Sterilisationsmedium wirkt für eine lange Zeit (vom Zuführen bis zum vollständigen Ausspülen) innerhalb der Dose gegen Mikroorganismen. Die verlängerte Einwirkzeit macht den Prozess besonders sicher und wirksam. Aufgrund der langen Einwirkzeit kann auch eine vergleichsweise geringe Konzentration des Sterilisationsmediums und/oder eine verringerte Menge des Sterilisationsmediums zum Innensterilisieren der Dosen verwendet werden. Vorteilhaft kann ferner auf besondere Maßnahmen zur Erhöhung der Wirksamkeit des Sterilisationsmediums verzichtet werden, wie beispielsweise auf eine Temperaturaktivierung und/oder auf ein Auskondensieren des Sterilisationsmediums innen an der Dose.

Das Verfahren kann vorteilhaft energetisch besonders effizient sein, da bspw. Medien eingespart werden. Durch das Entfallen eines separaten Prozessschrittes "Ausblasen des Sterilisationsmediums aus der Dose" kann je nach Maschinenleistung eine große Menge sterile Ausblasluft gespart werden. Daher kann vorteilhaft eine Prozesseinheit zur Herstellung steriler Luft verkleinert werden. Zusätzlich wird bspw. weniger sterile Druckluft benötigt.

Vorzugsweise kann das Spülen mittelbar oder unmittelbar vor dem Füllen oder unmittelbar vor einem Vorspannen der Dose in der Füllstation vor dem Füllen erfolgen.

In einem Ausführungsbeispiel ist das Sterilisationsmedium beim Zuführen und/oder beim Innensterilisieren gasförmig oder im Wesentlichen gasförmig. Alternativ oder zusätzlich kann das zugeführte Sterilisationsmedium derart zugeführt werden, dass es in der Dose nicht auskondensiert und/oder dass es in der Dose seinen gasförmigen Aggregatzustand beibehält. Vorteilhaft kann die vergleichsweise lange Einwirkzeit des Sterilisationsmediums ermöglichen, dass aufwändige Prozesstechnik zum Aktivieren und/oder Auskondensieren des Sterilisationsmediums eingespart werden kann.

In einem weiteren Ausführungsbeispiel verbleibt das zugeführte Sterilisationsmedium bis zu dem Spülen im Wesentlichen in der Dose. Vorteilhaft kann damit eine besonders wirksame Innensterilisation der Dose erreicht werden.

In einer Ausführungsform wird die Dose während des Zuführens des Sterilisationsmediums linear und/oder aufrechtstehend durch die Sterilisationsvorrichtung transportiert. Alternativ oder zusätzlich kann die Dose während des Innensterilisierens aufrechtstehend zu der Füllstation transportiert werden (z. B. mittels eines Dosenförderers). Vorteilhaft kann damit sichergestellt werden, dass möglichst wenig Sterilisationsmedium ungewünscht aus der Dose entweicht.

In einer weiteren Ausführungsform wird das Spülgas beim Spülen über einen, vorzugsweise zentralen, Spülgaszufuhrkanal der Füllstation zugeführt. Alternativ oder zusätzlich wird das Sterilisationsmedium beim Spülen in einen, vorzugsweise ringförmigen, Spülgasabfuhrkanal der Füllstation gespült.

In einer Ausführungsvariante weist das Verfahren ferner ein Absaugen des Sterilisationsmediums aus der Dose in der Füllstation, vorzugsweise beim Spülen der Dose in der Füllstation, auf.

In einer weiteren Ausführungsvariante erfolgt das Zuführen des Sterilisationsmediums mittels mindestens einer Düse der Sterilisationsvorrichtung. Vorteilhaft kann damit eine zielgenaue Zuführung des Sterilisationsmediums in die Dose hinein erreicht werden.

In einem Ausführungsbeispiel weist die mindestens eine Düse mindestens eine stationäre Düse auf, und die Dose bewegt sich während des Zuführens des Sterilisationsmediums an der mindestens einen stationären Düse vorbei. Vorteilhaft kann damit ein konstruktiv einfacher Aufbau der Sterilisationsvorrichtung ermöglicht werden.

In einem weiteren Ausführungsbeispiel weist die mindestens eine Düse mindestens eine bewegbare Düse auf. Vorzugsweise kann sich die mindestens eine bewegbare Düse während des Zuführens des Sterilisationsmediums mit der Dose mitbewegen. Vorteilhaft kann damit eine besonders zuverlässige und ausreichende Füllung der Dosen mit dem Sterilisationsmedium unterstützt werden. Vorteilhaft kann die mindestens eine bewegbare Düse gewährleisten, dass abhängig von einer Geometrie der Dose auch schwer zu erreichende Stellen der Dose von dem Sterilisationsmedium erreicht werden können.

In einer Ausführungsform ist eine Sterilisationsmedium-Austrittsmenge an der mindestens einen Düse konstant. Vorteilhaft kann damit ein Aufwand zum Steuern der Sterilisationsvorrichtung geringgehalten werden.

In einer weiteren Ausführungsform kann eine Sterilisationsmedium-Austrittsmenge an der mindestens einen Düse in Abhängigkeit von einer Transportgeschwindigkeit der Dose entlang der mindestens einen Düse angepasst werden (z. B. automatisch von einer Steuereinrichtung). Vorteilhaft kann damit eine ausreichende Füllung der Dose mit dem Sterilisationsmedium bei gleichzeitiger Einsparung von Sterilisationsmedium erreicht werden.

Vorzugsweise kann sich der Begriff "Steuereinrichtung" auf eine Elektronik (z. B. ausgeführt als eine Treiberschaltung oder mit Mikroprozessor(en) und Datenspeicher) und/oder eine mechanische, pneumatische und/oder hydraulische Steuerung beziehen, die je nach Ausbildung Steuerungsaufgaben und/oder Regelungsaufgaben und/oder Verarbeitungsaufgaben übernehmen kann. Auch wenn hierin der Begriff "Steuern" verwendet wird, kann damit gleichsam zweckmäßig auch "Regeln" bzw. "Steuern mit Rückkopplung" und/oder "Verarbeiten" umfasst bzw. gemeint sein.

In einer Ausführungsvariante wird das Sterilisationsmedium von der mindestens einen Düse gepulst abgegeben, vorzugsweise mit:
- einer Pulsfrequenz, die an eine Frequenz von sich an der mindestens einen Düse vorbeibewegenden Dosen angepasst ist; und/oder
- mit einer Pulsdauer, die an eine Transportgeschwindigkeit, mit der die Dose an der mindestens einen Düse vorbeibewegt wird, angepasst ist; und/oder
- mit einem oder mehreren Pulsen je Dose.

Vorteilhaft kann damit ebenfalls eine ausreichende Füllung der Dose mit dem Sterilisationsmedium bei gleichzeitiger Einsparung von Sterilisationsmedium erreicht werden.

In einer weiteren Ausführungsvariante weist die mindestens eine Düse auf:
- eine (z. B. stationäre oder bewegbare/sich bewegende) Düse, die das Sterilisationsmedium senkrecht von oben und mittig in die Dose zuführt (z. B. einbläst); und/oder
- eine (z. B. stationäre oder bewegbare/sich bewegende) Düse, die das Sterilisationsmedium senkrecht von oben und außermittig in die Dose zuführt (z. B. einbläst); und/oder
- eine (z. B. stationäre oder bewegbare/sich bewegende) Düse, die das Sterilisationsmedium schräg angewinkelt zu einer Hochachse der Dose von oben in die Dose zuführt (z. B. einbläst), bevorzugt angewinkelt zwischen 10° und 20° zu der Hochachse, besonders bevorzugt angewinkelt um 15° zu der Hochachse.

Vorteilhaft kann damit eine besonders gute Füllung der Dose mit dem Sterilisationsmittel erreicht werden.

In einem Ausführungsbeispiel weist das Verfahren ferner ein Sterilisieren mindestens eines Abschnitts der Füllstation mittels des aus der Dose ausgespülten Sterilisationsmediums (z. B. direkt beim Ausspülen aus dem Behälter), wobei vorzugsweise der mindestens eine Abschnitt einen Füllgutauslass der Füllstation und/oder eine Dichtfläche der Füllstation aufweist, auf. Vorteilhaft kann das Sterilisationsmedium somit doppelt genutzt werden, nämlich primär zum Innensterilisieren der Behälter und sekundär zum Sterilisieren von Teilen der Füllstation beim Ausspülen aus dem Behälter.

In einem weiteren Ausführungsbeispiel wird die gefüllte Dose in der Dosenbehandlungsanlage nicht pasteurisiert.

Ein weiterer Aspekt der vorliegenden Offenbarung betrifft eine Dosenbehandlungsanlage, die vorzugsweise zum Ausführen eines Verfahrens wie hierin offenbart ausgebildet ist. Die Dosenbehandlungsanlage weist eine Sterilisationsvorrichtung auf, die zum Zuführen eines, vorzugsweise gasförmigen, Sterilisationsmediums, vorzugsweise aufweisend Wasserstoffperoxid, in Dosen ausgebildet ist. Die Dosenbehandlungsanlage weist eine Dosenfüllvorrichtung (z. B. Dosenfüllerkarussell) auf, die (dosen-) stromabwärts von der Sterilisationsvorrichtung angeordnet ist und mindestens eine Füllstation, die zum Spülen der Dosen zum Ausspülen des Sterilisationsmediums aus den Dosen und zum Füllen der Dosen mit einem Füllgut ausgebildet ist, aufweist. Vorteilhaft kann die Dosenbehandlungsanlage die gleichen Vorteile erzielen, die bereits unter Bezugnahme auf das Verfahren erläutert wurden.

In einem Ausführungsbeispiel weist die Dosenbehandlungsanlage ferner einen Dosenförderer auf, der die Sterilisationsvorrichtung direkt mit der Dosenfüllvorrichtung verbindet. Alternativ oder zusätzlich kann die Dosenbehandlungsanlage frei von einer Pasteurisiervorrichtung zum Pasteurisieren der Dosen nach dem Füllen sein. Alternativ oder zusätzlich kann die Dosenbehandlungsanlage vom Beginn der Sterilisationsvorrichtung bis hin zur Dosenfüllvorrichtung keine Vorrichtung aufweisen, die zum Entfernen des Sterilisationsmediums aus den Dosen ausgebildet ist.

Vorzugsweise kann die Sterilisationsvorrichtung mindestens eine Düse zum Zuführen des Sterilisationsmediums zu den Dosen aufweisen.

Bevorzugt kann die mindestens eine Düse mindestens eine stationäre Düse und/oder mindestens eine bewegbare Düse aufweisen.

In einer Weiterbildung kann die mindestens eine Düse aufweisen:
eine (z. B. stationäre oder bewegbare/sich bewegende) Düse, die so ausgerichtet, ausgebildet und/oder bewegbar ist, dass das Sterilisationsmedium senkrecht von oben und mittig in die jeweilige Dose zugeführt (z. B. eingeblasen) wird; und/oder
eine (z. B. stationäre oder bewegbare/sich bewegende) Düse, die so ausgerichtet, ausgebildet und/oder bewegbar ist, dass das Sterilisationsmedium senkrecht von oben und außermittig in die jeweilige Dose zugeführt (z. B. eingeblasen) wird; und/oder
eine (z. B. stationäre oder bewegbare/sich bewegende) Düse, die so ausgerichtet, ausgebildet und/oder bewegbar ist, dass das Sterilisationsmedium schräg angewinkelt zu einer Hochachse der jeweiligen Dose von oben in die jeweilige Dose zugeführt (z. B. eingeblasen) wird, bevorzugt angewinkelt zwischen 10° und 20° zu der Hochachse, besonders bevorzugt angewinkelt um 15° zu der Hochachse.

Optional kann die Sterilisationsvorrichtung einen Dosenförderer zum Transportieren der Dosen während des Zuführens des Sterilisationsmediums zu den Dosen aufweisen. Bevorzugt ist der Dosenförderer dazu ausgebildet, die Dosen aufrechtstehend und/oder linear durch die Sterilisationsvorrichtung zu transportieren.

Beispielsweise kann die Sterilisationsvorrichtung ein (z. B. tunnelförmiges oder blockförmiges) Gehäuse aufweisen, in dem die mindestens eine Düse und/oder der Dosenförderer angeordnet sind.

Die zuvor beschriebenen bevorzugten Ausführungsformen und Merkmale der Erfindung sind beliebig miteinander kombinierbar.

### Kurzbeschreibung der Figuren

Weitere Einzelheiten und Vorteile der Erfindung werden im Folgenden unter Bezug auf die beigefügten Zeichnungen beschrieben. Es zeigen:
- Figur 1: eine schematische Darstellung einer Dosenbehandlungsanlage gemäß einem Ausführungsbeispiel der vorliegenden Offenbarung;
- Figur 2: eine schematische, perspektivische Schnittansicht durch eine Sterilisationsvorrichtung;
- Figur 3: eine weitere schematische, perspektivische Schnittansicht der Sterilisationsvorrichtung von Figur 2; und
- Figur 4: eine Schnittansicht eines Bereichs einer Füllstation.

Die in den Figuren gezeigten Ausführungsformen stimmen zumindest teilweise überein, so dass ähnliche oder identische Teile mit den gleichen Bezugszeichen versehen sind und zu deren Erläuterung auch auf die Beschreibung der anderen Ausführungsformen bzw. Figuren verwiesen wird, um Wiederholungen zu vermeiden.

### Detaillierte Beschreibung beispielhafter Ausführungsformen

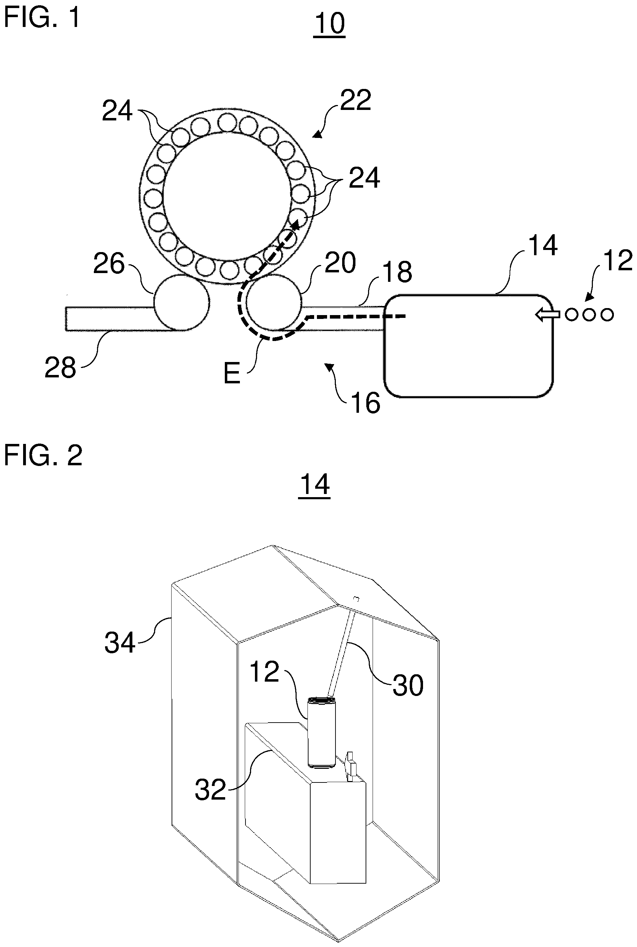

Die Figur 1 zeigt eine Dosenbehandlungsanlage 10, also eine Anlage 10 zum Behandeln von Dosen 12. Die Dosen 12 können beispielsweise Getränkedosen oder Konservendosen sein. Die Dosenbehandlungsanlage 10 kann als eine Dosenabfüllanlage ausgeführt sein. Bevorzugt werden die Dosen 12 in der Dosenbehandlungsanlage 10 im sogenannten Basehandling gehandhabt und transportiert.

Die Dosenbehandlungsanlage 10 weist eine Sterilisationsvorrichtung 14 und eine Dosenfüllvorrichtung 22 mit mindestens einer Füllstation 24 auf. Die Sterilisationsvorrichtung 14 kann auch als eine Entkeimungsvorrichtung bezeichnet werden. Die Dosenbehandlungsanlage 10 kann ferner einen Dosenförderer 16 aufweisen.

Die Sterilisationsvorrichtung 14 führt ein Sterilisationsmedium in die Dosen 12 ein bzw. zu. Beispielsweise kann die Sterilisationsvorrichtung 14 das Sterilisationsmedium in die Dosen 12 eindüsen. Das Sterilisationsmedium kann auch als Entkeimungsmedium bezeichnet werden.

Bevorzugt können die Dosen 12 während des Zuführens des Sterilisationsmediums linear durch die Sterilisationsvorrichtung 14 transportiert werden. Bevorzugt können die Dosen 12 während des Zuführens des Sterilisationsmediums aufrechtstehend durch die Sterilisationsvorrichtung 14 transportiert werden.

Das von der Sterilisationsvorrichtung 14 zugeführte Sterilisationsmedium ist bevorzugt gasförmig oder zumindest im Wesentlichen gasförmig. Das zugeführte Sterilisationsmedium kann die Dosen 12 füllen. Das Sterilisationsmedium kann in den Dosen 12 bspw. schwerkraftbedingt und aufgrund einer aufrechten Ausrichtung der Dosen 12 verbleiben.

Das zugeführte Sterilisationsmedium kann von der Sterilisationsvorrichtung 14 vorzugsweise derart zugeführt werden, dass es in der Dose 12 nicht auskondensiert und dass es in der Dose 12 seinen (im Wesentlichen) gasförmigen Aggregatzustand beibehält. Beispielsweise können die Dosen 12 vor, beim und/oder nach dem Zuführen eine Temperatur aufweisen, die der Umgebungstemperatur entspricht. Es ist möglich, dass das Sterilisationsmedium mit einer Temperatur zu der Dose 12 zugeführt wird, die ein Auskondensieren des Sterilisationsmediums in der Dose 12 verhindert und/oder ein Beibehalten des (im Wesentlichen) gasförmigen Aggregatzustands des Sterilisationsmediums in der Dose 12 unterstützt. Es ist ebenfalls möglich, dass die Dosen 12 und/oder das Sterilisationsmedium beispielsweise vor dem Zuführen des Sterilisationsmediums vorgewärmt und/oder nach dem Zuführen des Sterilisationsmediums nachgewärmt wird/werden.

Bevorzugt kann das Sterilisationsmedium Wasserstoffperoxid sein oder aufweisen. Bevorzugt wird das Sterilisationsmedium vor dem Zuführen verdampft. Das Sterilisationsmedium kann beispielsweise als ein Gemisch mit Luft zu den Dosen 12 zugeführt werden.

Das zugeführte Sterilisationsmedium sterilisiert die Dosen 12 innen, während die Dosen 12 zu den Füllstationen 24 transportiert werden. Vorzugsweise bleibt das Sterilisationsmedium während des Innensterilisieren gasförmig oder zumindest im Wesentlichen gasförmig.

Beispielsweise können die Dosen 12 nach dem Zuführen des Sterilisationsmediums direkt oder über den Dosenförderer 16 zum den Füllstationen 24 transportiert werden. Von der Sterilisationsvorrichtung 14 zu der jeweiligen Füllstation 24 können die Dosen 12 vorzugsweise aufrechtstehend transportiert werden.

Bevorzugt weist die Dosenbehandlungsanlage 10 bezüglich eines Dosenstroms durch die Dosenbehandlungsanlage 10 vom Beginn der Sterilisationsvorrichtung 14 bis hin zur Dosenfüllvorrichtung 22 keine Vorrichtung auf, die zum Entfernen des Sterilisationsmediums aus den Dosen 12 ausgebildet ist.

Das zugeführte Sterilisationsmedium kann die Dosen 12 während des Transports entlang einer Einwirkstrecke E innensterilisieren. Die Einwirkstrecke E kann sich beispielsweise ausgehend von einer Position in der Sterilisationsvorrichtung 14, an der das Sterilisationsmedium zu der jeweiligen Dose 12 zugeführt wird, erstrecken. Die Einwirkstrecke E kann sich bis hin zu der jeweiligen Füllstation 24 an einer Position, an der die jeweilige Füllstation 24 beginnt, die jeweilige Dose 12 mit einem Spülgas zu spülen, erstrecken. Entlang der Einwirkstrecke E bzw. bis zum Spülen in der Füllstation 24 kann das Sterilisationsmedium im Wesentlichen in der Dose 12 verbleiben und wirken.

Der Dosenförderer 16 kann die Sterilisationsvorrichtung 14 direkt mit der Dosenfüllvorrichtung 22 verbinden. Der Dosenförderer 16 kann bezüglich eines Dosenstroms durch die Dosenbehandlungsanlage 10 direkt stromabwärts von der Sterilisationsvorrichtung 14 angeordnet sein. Der Dosenförderer 16 kann bezüglich eines Dosenstroms durch die Dosenbehandlungsanlage 10 direkt stromaufwärts von der Dosenfüllvorrichtung 22 angeordnet sein.

Der Dosenförderer 16 kann die Dosen 12 vorzugsweise aufrechtstehend, zum Beispiel im Basehandling, transportieren.

Der Dosenförderer 16 kann beispielsweise einen Linearförderer 18 und/oder einen Einlaufstern 20 aufweisen. Bspw. kann der Linearförderer 18 die Dosen 12 von der Sterilisationsvorrichtung 14 übernehmen und an den Einlaufstern 20 übergeben. Der Einlaufstern 20 kann die Dosen 12 nacheinander an die Füllstationen 24 übergeben. Der Einlaufstern 20 kann die Dosen beispielsweise direkt von der Sterilisationsvorrichtung 14 oder von dem Linearförderer 18 übernehmen.

Die Dosenfüllvorrichtung 22 ist bezüglich eines Dosenstroms durch die Dosenbehandlungsanlage 10 (dosen-) stromabwärts von der Sterilisationsvorrichtung 14 angeordnet. Beispielsweise kann die Dosenfüllvorrichtung 22 direkt stromabwärts von der Sterilisationsvorrichtung 14 angeordnet sein. Alternativ kann die Dosenfüllvorrichtung 22 beispielsweise direkt stromabwärts von dem Dosenförderer 16, der wiederum direkt stromabwärts von der Sterilisationsvorrichtung 14 angeordnet sein kann, angeordnet sein.

Die Dosenfüllvorrichtung 22 weist bevorzugt mehrere Füllstationen 24 auf. In den mehreren Füllstationen 24 kann vorzugsweise jeweils eine Dose 12 behandelt werden. In den mehreren Füllstationen 24 können somit gleichzeitig oder zumindest zeitlich überlappend mehrere der Dosen 12 behandelt werden.

Bevorzugt kann die Dosenfüllvorrichtung 22 als ein Dosenfüllerkarussell bzw. ein Rundläufer-Dosenfüller ausgebildet sein. Die Füllstationen 24 können verteilt um einen Umfang der Dosenfüllvorrichtung 22 angeordnet sein. Es ist jedoch auch möglich, dass die Dosenfüllvorrichtung 22 beispielsweise ein Lineardosenfüller mit mehreren in Reihe hintereinander und/oder nebeneinander angeordneten Füllstationen ist (nicht in Figur 1 dargestellt).

Die Füllstation 24 ist zum Spülen einer jeweiligen Dose 12 zum Ausspülen des Sterilisationsmediums aus der Dose 12 ausgebildet. Das Spülen kann beispielsweise unmittelbar vor dem Füllen oder unmittelbar vor einem Vorspannen vor dem Füllen erfolgen.

Im Einzelnen spült die Füllstation 24 die jeweilige Dose 12 mit einem Spülgas, wodurch das Sterilisationsmedium nach dem Innensterilisieren der Dose 12 aus der Dose 12 ausgespült wird. Beim Spülen kann auch Sauerstoff aus der Dose 12 ausgespült werden. Das Spülgas ist vorzugsweise ein Prozessgas der Dosenfüllvorrichtung 22. Bevorzugt ist das Spülgas Kohlenstoffdioxid.

Es ist möglich, dass das Spülen der Dose 12, d.h. das Ausspülen des Sterilisationsmediums und ggf. von Sauerstoff beim Spülen, in der Füllstation 24 zusätzlich durch ein Absaugen des Sterilisationsmediums mittels der Füllstation 24 unterstützt wird.

Es ist auch möglich, dass das aus der Dose 12 ausgespülte Sterilisationsmedium mindestens einen Bereich der Füllstation 24 beim Ausströmen aus der Dose 12 sterilisiert. Beispielsweise kann das Sterilisationsmedium auf seinem Weg durch die Füllstation 24 einen Füllgutauslass der Füllstation 24 und/oder Dichtflächen der Füllstation 24 passieren und dabei sterilisieren.

Alternativ oder zusätzlich kann das ausgespülte Sterilisationsmedium bspw. gesammelt und wiederverwendet werden, z. B. von der Sterilisationsvorrichtung 14.

Die Füllstation 24 ist zudem zum Füllen der Dosen 12 mit einem Füllgut ausgebildet. Die Füllstation 24 füllt die jeweilige Dose 12 nach dem Spülen, zum Beispiel unmittelbar nach dem Spülen oder nach einem Vorspannen der Dose 12. Das Füllgut ist vorzugsweise ein flüssiges oder pastöses Füllgut, zum Beispiel ein Getränk oder ein Nahrungsmittel. Die Füllstation 24 kann bspw. eine aseptische Füllstation zum aseptischen Füllen der Dosen 12 mit dem Füllgut sein.

Es ist möglich, dass die Füllstation 24 die jeweilige Dose 12 zudem evakuieren kann, z. B. vor oder nach dem Spülen, und/oder vorspannen kann, z. B. nach dem Spülen.

Nach dem Füllen der Dosen 12 in der Dosenfüllvorrichtung 22 können die Dosen 12 bspw. zu einer Verschließvorrichtung weitertransportiert werden (nicht in Figur 1 dargestellt). Die Verschließvorrichtung kann die gefüllten Dosen 12 mit einem Dosendeckel verschließen. Die Verschließvorrichtung kann zum Verschließen der Dosen beispielsweise mindestens einen Verschließerkopf aufweisen. Bevorzugt kann die Verschließvorrichtung als ein Verschließerkarussell bzw. ein Rundläufer-Verschließer ausgebildet sein.

Besonders bevorzugt ist es nicht notwendig und daher auch nicht vorgesehen, dass die gefüllten Dosen 12 in der Dosenbehandlungsanlage 10 noch pasteurisiert werden. Die Dosenbehandlungsanlage 10 kann entsprechend frei von einer Pasteurisiervorrichtung zum Pasteurisieren der Dosen 12 nach dem Füllen sein.

Es ist möglich, dass die Dosenbehandlungsanlage 10 weitere Dosenbehandlungsvorrichtungen aufweist, wie bspw. eine Entpalletiervorrichtung und/oder eine Rinsvorrichtung (z. B. mit Rinsdüsen), die bspw. (dosen-) stromaufwärts von der Sterilisationsvorrichtung 14 angeordnet sein kann/können. Alternativ oder zusätzlich kann Dosenbehandlungsanlage 10 beispielsweise die bereits erwähnte Verschließvorrichtung, eine Klebegebindeherstellvorrichtung (z. B. mit Klebstoffapplikationsdüsen), eine Verpackungsvorrichtung (z. B. mit Packköpfen oder Folieneinschlägern) und/oder eine Palettiervorrichtung (z. B. mit Palettierköpfen, Schiebeplatten oder dgl.) aufweisen., die bspw. (dosen-) stromabwärts von der Dosenfüllvorrichtung 22 angeordnet sein kann/können.

Es ist ebenfalls möglich, dass die Dosenbehandlungsanlage 10 weitere Dosenförderer zum Transportieren der Dosen 12 durch die Dosenbehandlungsanlage 10 aufweist. Die weiteren Dosenförderer können beispielsweise mindestens einen Transportstern und/oder mindestens einen Linearförderer aufweisen. Beispielsweise kann ein Auslaufstern 26 die von der Dosenfüllvorrichtung 22 gefüllten Dosen 12 übernehmen und an einen nachgeordneten Dosenförderer 28, zum Beispiel einen Linearförderer, zum Weitertransport übergeben.

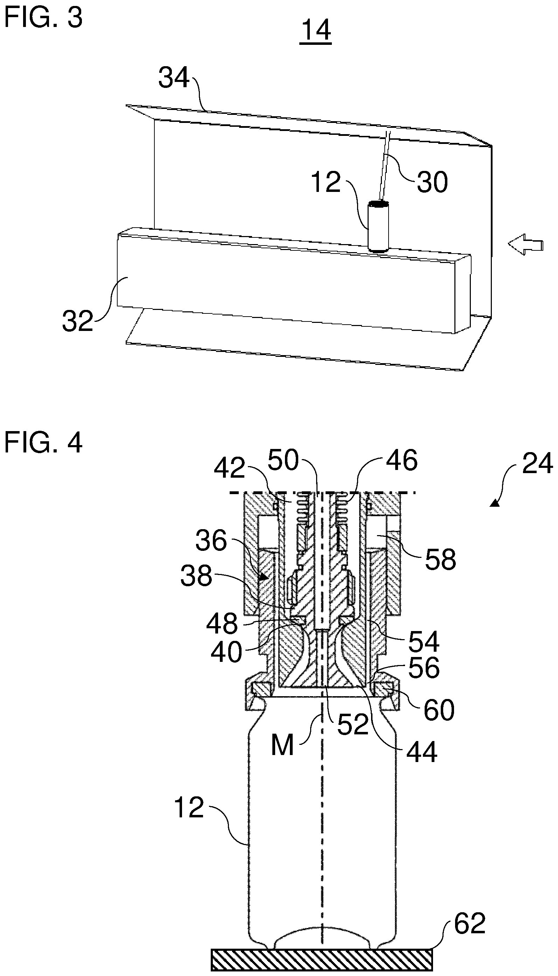

Die Figuren 2 und 3 zeigen eine beispielhafte Ausführungsform der Sterilisationsvorrichtung 14.

Die Sterilisationsvorrichtung 14 kann beispielsweise mindestens eine Düse 30, einen Dosenförderer 32 und/oder ein Gehäuse 34 aufweisen.

Die mindestens eine Düse 30 kann das Sterilisationsmedium zu den Dosen 12 zuführen bzw. in die Dosen 12 einführen/einfüllen. Vorzugsweise taucht die mindestens eine Düse 30 beim Zuführen des Sterilisationsmediums zu der jeweiligen Dose 12 nicht in die jeweilige Dose 12 ein.

Die mindestens eine Düse 30 kann mindestens eine stationäre Düse aufweisen. Die jeweilige Dose 12 kann sich während des Zuführens des gasförmigen Sterilisationsmediums aus der stationären Düse an der stationären Düse vorbeibewegen.

Die mindestens eine Düse 30 kann mindestens eine bewegbare Düse aufweisen. Die bewegbare Düse kann sich während des Zuführens des Sterilisationsmediums aus der bewegbaren Düse mit der jeweiligen Dose 12 mitbewegen.

Die bewegbare Düse kann beispielsweise verschwenkbar sein, um der sich bewegenden jeweiligen Dose 12 zu folgen. Alternativ oder zusätzlich kann die bewegbare Düse beispielsweise verschiebbar sein, um der sich bewegenden jeweiligen Dose 12 zu folgen. Die verschiebbare Düse kann sich beispielsweise hin und her bewegen oder entlang einer geschlossenen Bahn bewegen.

Die Sterilisationsvorrichtung 14 kann mindestens einen Antrieb, zum Beispiel einen Elektromotor, zum Antreiben einer Bewegung der bewegbaren Düse aufweisen. Es ist möglich, dass der Antrieb zum Antreiben der Bewegung der bewegbaren Düse mit einem Antrieb des Dosenförderers 32 gekoppelt ist. Es ist auch möglich, dass der Antrieb zum Antreiben der Bewegung der bewegbaren Düse auch den Dosenförderer 32 antreibt.

Die Sterilisationsvorrichtung 14 kann ferner mindestens eine Führung zum Führen der Bewegung der bewegbaren Düse aufweisen. Die mindestens Einführung kann beispielsweise eine Schwenkführung, eine Linearführung und/oder eine Bahnführung aufweisen.

Die mindestens eine Düse 30 kann bspw. eine Düse aufweisen, die das Sterilisationsmedium senkrecht von oben und mittig in die Dose 12 einbläst. Zusätzlich oder alternativ kann die mindestens eine Düse 30 bspw. eine Düse aufweisen, die das Sterilisationsmedium senkrecht von oben und außermittig in die Dose 12 einbläst. Zusätzlich oder alternativ kann die mindestens eine Düse 30 bspw. eine Düse aufweisen, die das Sterilisationsmedium schräg angewinkelt zu einer Hochachse der Dose 12 von oben einbläst. Vorzugsweise kann ein von der Düse ausgehende Sterilisationsmedium-Strahl in einem Winkel zwischen 10° und 20°, besonders bevorzugt rd. 15°, angewinkelt zu einer Hochachse der Dose 12 sein.

Die mindestens eine Düse 30 kann bspw. eine konstante Sterilisationsmedium-Austrittsmenge ausgeben. Alternativ kann die Sterilisationsmedium-Austrittsmenge in Abhängigkeit von einer Transportgeschwindigkeit der Dose 12 bzw. des Dosenförderers 32 entlang der mindestens einen Düse 30 angepasst werden, z. B. automatisch von einer Steuereinrichtung.

Es ist möglich, dass das Sterilisationsmedium von der mindestens einen Düse 30 gepulst abgegeben wird, z. B. mit einem oder mehreren Pulsen je Dose 12. Vorzugsweise kann eine Pulsfrequenz an eine Frequenz von sich an der mindestens einen Düse 30 vorbeibewegenden Dosen 12 angepasst sein. Bevorzugt kann eine Pulsdauer an eine Transportgeschwindigkeit (z. B. des Dosenförderers 32), mit der die Dose 12 an der mindestens einen Düse 30 vorbeibewegt wird, angepasst sein.

Der Dosenförderer 32 kann die Dosen 12 durch die Sterilisationsvorrichtung 14 transportieren. Der Dosenförderer 32 ist bevorzugt ein Linearförderer. Der Linearförderer kann die Dosen 12 linear durch die Sterilisationsvorrichtung 14 transportieren.

Vorzugsweise können die Dosen 12 aufrechtstehend von den Dosenförderer 32 transportiert werden. Der Dosenförderer 32 kann die Dosen 12 beispielsweise im Basehandling transportieren. Beispielsweise kann der Dosenförderer 32 ein Bandförderer, ein Mattenkettenförderer oder ein Plattenförderer sein.

Der Dosenförderer 32 kann die Dosen 12 beispielsweise einspurig, mehrspurig oder im ungeordneten Massenstrom durch die Sterilisationsvorrichtung 14 transportieren.

Die mindestens eine Düse 30 und/oder der Dosenförderer 32 können in dem Gehäuse 34 der Sterilisationsvorrichtung 14 angeordnet sein. Das Gehäuse 34 kann beispielsweise eine Tunnelform oder eine Blockform aufweisen.

Die Figur 4 zeigt einen Bereich einer beispielhaften Ausführungsform der Füllstation 24.

Die Füllstation 24 kann bspw. ein Füllventil 36, einen Spülgaszufuhrkanal 50, einen Spülgasabfuhrkanal 54, eine Dichtung 60 und/oder eine Dosenabstützung 62 aufweisen.

Das Füllventil 36 kann die unter dem Füllventil 36 positionierte Dose 12 mit dem Füllgut füllen. Das Füllventil 36 kann ein bewegbares Ventilglied 38 zum Öffnen und Schließen des Füllventils 24 aufweisen.

Das Ventilglied 38 kann entlang einer Mittelachse M des Füllventils 36 bzw. der Füllstation 24 bewegbar sein. Die Mittelachse M kann eine Mittellängsachse (Mittelhochachse) des Füllventils 36 bzw. der Füllstation 24 sein. Das Ventilglied 38 kann gegenüber einem, vorzugsweise trichterförmigen, Ventilsitz 40 des Füllventils 36 bewegbar sein. Vorzugsweise kann der Ventilsitz 40 ein Ventilkegelsitz sein.

In einer Öffnungsstellung kann das Ventilglied 38 eine Fluidverbindung zwischen einer Füllgutkammer 42 und einem Füllgutauslass 44 des Füllventils 36 freigeben. Bspw. kann eine Füllgutzufuhrleitung (nicht dargestellt in Figur 4) mit der Füllgutkammer 42 zum Zuführen von Füllgut zu der Füllgutkammer 42 verbunden sein. Das Füllgut kann von der Füllgutkammer 42 zu einem Ringspalt zwischen dem Ventilglied 38 und dem Ventilsitz 40 und von dort zu dem Füllgutauslass 44 strömen.

Der Füllgutauslass 44 kann beispielsweise als ein Ringspalt ausgeführt sein. Der Ringspalt kann bspw. innenumfangsseitig von dem Ventilglied 38 begrenzt sein. Der Füllgutauslass 44 kann koaxial zu der Mittelachse M angeordnet sein. Bevorzugt kann sich das Ventilglied 38 im Bereich des Füllgutauslasses 44 hin zu einem freien Ende des Ventilglieds 38 trichterförmig erweitern.

Das Ventilglied 38 kann sich durch die Füllgutkammer 42 erstrecken. Das Ventilglied 38 kann gegenüber der Füllgutkammer 42 mit einem Faltenbalg 46 abgedichtet sein.

In einer Schließstellung kann das Ventilglied 38 die Fluidverbindung zwischen der Füllgutkammer 42 und dem Füllgutauslass 44 des Füllventils 36 blockieren, wie beispielhaft in Figur 4 dargestellt ist. Das Füllgut aus der Füllgutkammer 42 kann das am Ventilsitz 40 abdichtend anliegende Ventilglied 38 nicht passieren.

Beispielsweise kann das Ventilglied 38 mittels eines elastischen Elements (z. B. eine Feder, vorzugsweise eine Schraubendruckfeder) in einer Richtung entlang der Mittelachse M vorgespannt sein, z. B. zum Öffnen oder zum Schließen des Füllventils 36. Bevorzugt kann das Ventilglied 38 mittels eines Aktors in einer Richtung entlang der Mittelachse M entgegen der elastischen Vorspannung bewegbar sein, z. B. zum Öffnen oder zum Schließen des Füllventils 36. Beispielsweise kann der Aktor ein Pneumatikaktor oder ein elektromagnetischer Aktor sein.

Das Ventilglied 38 kann ein, vorzugsweise ringförmiges, Dichtelement 48 zum Abdichten zu dem Ventilsitz 40 aufweisen. Bevorzugt kann das Dichtelement 48 an einem Kegelabschnitt des Ventilglieds 38 angeordnet sein.

Der Spülgaszufuhrkanal 50 kann sich parallel zu der Mittelachse M erstrecken. Bevorzugt kann sich der Spülgaszufuhrkanal 50 durch das Ventilglied 38 hindurch erstrecken.

An einem oberen Endbereich des Ventilglieds 38 kann ein Gaseinlass des Spülgaszufuhrkanals 50 angeordnet sein. Der Gaseinlass kann beispielsweise in Fluidverbindung mit einem Spülventil und/oder mit einem Vorspannventil sein (beide nicht in den Figur 4 dargestellt).

An einem unteren Endbereich des Ventilglieds 38 kann ein Gasauslass 52 des Spülgaszufuhrkanals 50 angeordnet sein. Beispielsweise kann der Füllgutauslass 44 sich ringförmig um den Gasauslass 52 herum erstrecken. Über den Spülgaszufuhrkanal 50 kann der Dose 12 ein verdichtetes Spülgas und optional ein Vorspanngas zugeführt werden. Das Spülgas kann das in der Dose 12 befindliche Sterilisationsmedium sowie Luft mit Sauerstoff aus der Dose 12 ausspülen.

Der Spülgasabfuhrkanal 54 kann sich parallel zu der Mittelachse M erstrecken. Bevorzugt kann sich der Spülgasabfuhrkanal 54 als ein Ringkanal erstrecken.

Ein Gaseinlass 56 des Spülgasabfuhrkanals 54 kann an einem unteren Ende des Spülgasabfuhrkanals 54 angeordnet sein. Beispielsweise kann sich der Gaseinlass 56 ringförmig um den Füllgutauslass 44 herum erstrecken.

Der Spülgasabfuhrkanal 54 kann an seinem oberen Ende in einen Gaskanal 58 münden. Über den Gaskanal 58 kann Gas aus der Dose 12 entweichen bzw. abgeführt werden, z. B. das Sterilisationsmedium, Spülgas, Vorspanngas und/oder Restgas. Entsprechend kann der Gaskanal 58 beispielsweise in Fluidverbindung mit einer Abführleitung und/oder einem Abführventil sein (beide nicht in Figur 4 dargestellt).

Die Dichtung 60 kann zwischen der Füllstation 24 und einer umlaufenden Oberkante (z. B. Bördelrand) der Dose 12 abdichten. Während die Dichtung 60 an der Oberkante der Dose 12 anliegt, kann die Dose 12 gespült und gefüllt werden, sowie bspw. evakuiert und/oder vorgespannt. Die Dichtung 60 kann beispielsweise als ein Dichtring, zum Beispiel ein O-Ring, ausgebildet sein.

Die Dichtung 60 kann in einer Dichtungsaufnahme der Füllstation 24, vorzugsweise kraft- und/oder formschlüssig, aufgenommen und gesichert sein. Die Dichtungsaufnahme ist bevorzugt ringförmig. Die Dichtungsaufnahme kann als eine Nut ausgebildet sein. Die Dichtung 60 kann sich koaxial um die Mittelachse M erstrecken.

Beim Ausspülen des Sterilisationsmedium aus dem Behälter der Dose 12 kann das Sterilisationsmedium beispielsweise noch den Füllgutauslass 44 und/oder einen Abschnitt der Dichtung 60 passieren und dabei sterilisieren.

Die Dosenabstützung 62 kann die Dose 12 abstützen. Die Dosenabstützung 62 kann beispielsweise eine Stützplatte aufweisen, die die Dose 12 bodenseitig abstützt. Alternativ oder zusätzlich kann die Dosenabstützung 62 beispielsweise eine Dosenhalterung aufweisen, die die Dose 12 an einer Mantelfläche der Dose 12 abstützt (nicht in den Figuren dargestellt). Die Dosenhalterung kann beispielsweise als Klammer, Greifer oder Tasche ausgeführt sein.

Vorzugsweise kann die Füllstation 24 eine Hubeinrichtung zum vertikalen Bewegen des Füllventils 24 und/oder eine Hubeinrichtung zum vertikalen Bewegen der Dosenabstützung 62 aufweisen. Entsprechend können die Dose 12 und die Füllstation 24 eine relative Hubbewegung zueinander ausführen, um die Füllstation 24 und die Dose 12 vor dem Behandeln aneinander anzunähern und ggf. aneinander anzupressen.

Die Erfindung ist nicht auf die vorstehend beschriebenen bevorzugten Ausführungsbeispiele beschränkt. Vielmehr ist eine Vielzahl von Varianten und Abwandlungen möglich, die ebenfalls von dem Erfindungsgedanken Gebrauch machen und deshalb in den Schutzbereich fallen. Insbesondere beansprucht die Erfindung auch Schutz für den Gegenstand und die Merkmale der Unteransprüche unabhängig von den in Bezug genommenen Ansprüchen. Insbesondere sind die einzelnen Merkmale des unabhängigen Anspruchs 1 jeweils unabhängig voneinander offenbart. Zusätzlich sind auch die Merkmale der Unteransprüche unabhängig von sämtlichen Merkmalen des unabhängigen Anspruchs 1 offenbart. Alle Bereichsangaben hierin sind derart offenbart zu verstehen, dass gleichsam alle in den jeweiligen Bereich fallenden Werte einzeln offenbart sind, z. B. auch als jeweils bevorzugte engere Außengrenzen des jeweiligen Bereichs.

### Bezugszeichenliste

- 10: Dosenbehandlungsanlage
- 12: Dose
- 14: Sterilisationsvorrichtung
- 16: Dosenförderer
- 18: Linearförderer
- 20: Einlaufstern
- 22: Dosenfüllvorrichtung
- 24: Füllstation
- 26: Auslaufstern
- 28: Dosenförderer
- 30: Düse
- 32: Dosenförderer
- 34: Gehäuse
- 36: Füllventil
- 38: Ventilglied
- 40: Ventilsitz
- 42: Füllgutkammer
- 44: Füllgutauslass
- 46: Faltenbalg
- 48: Dichtelement
- 50: Spülgaszufuhrkanal
- 52: Gasauslass
- 54: Spülgasabfuhrkanal
- 56: Gaseinlass
- 58: Gaskanal
- 60: Dichtung
- 62: Dosenabstützung

- E: Einwirkstrecke
- M: Mittelachse

## Patentansprüche

1. Verfahren zum Behandeln von Dosen (12) in einer Dosenbehandlungsanlage (10), wobei das Verfahren aufweist:
Zuführen eines Sterilisationsmediums, vorzugsweise aufweisend Wasserstoffperoxid, in eine Dose (12) mittels einer Sterilisationsvorrichtung (14);
Innensterilisieren der Dose (12) durch das zugeführte Sterilisationsmedium während die Dose (12) zu einer Füllstation (24) transportiert wird;
Spülen der Dose (12) in der Füllstation (24) mit einem Spülgas, vorzugsweise Kohlenstoffdioxid, wodurch das Sterilisationsmedium aus der Dose (12) ausgespült wird; und
Füllen der gespülten Dose (12) in der Füllstation (24) mit einem Füllgut.

2. Verfahren nach Anspruch 1, wobei mindestens eines erfüllt ist von:
das Sterilisationsmedium ist beim Zuführen und/oder beim Innensterilisieren gasförmig oder im Wesentlichen gasförmig;
das zugeführte Sterilisationsmedium wird derart zugeführt, dass es in der Dose (12) nicht auskondensiert und/oder dass es in der Dose (12) seinen gasförmigen Aggregatzustand beibehält; und
das zugeführte Sterilisationsmedium verbleibt bis zu dem Spülen im Wesentlichen in der Dose (12).

3. Verfahren nach Anspruch 1 oder Anspruch 2, wobei:
die Dose (12) während des Zuführens des Sterilisationsmediums linear und/oder aufrechtstehend durch die Sterilisationsvorrichtung (14) transportiert wird; und/oder
die Dose (12) während des Innensterilisierens aufrechtstehend zu der Füllstation (24) transportiert wird.

4. Verfahren nach einem der vorherigen Ansprüche, wobei:
das Spülgas beim Spülen über einen, vorzugsweise zentralen, Spülgaszufuhrkanal (50) der Füllstation (24) zugeführt wird; und/oder
das Sterilisationsmedium beim Spülen in einen, vorzugsweise ringförmigen, Spülgasabfuhrkanal (54) der Füllstation (24) gespült wird.

5. Verfahren nach einem der vorherigen Ansprüche, ferner aufweisend:
Absaugen des Sterilisationsmediums aus der Dose (12) in der Füllstation (24), vorzugsweise beim Spülen der Dose (12) in der Füllstation (24).

6. Verfahren nach einem der vorherigen Ansprüche, wobei:
das Zuführen des Sterilisationsmediums mittels mindestens einer Düse (30) der Sterilisationsvorrichtung (14) erfolgt.

7. Verfahren nach Anspruch 6, wobei:
die mindestens eine Düse (30) mindestens eine stationäre Düse aufweist, und die Dose (12) sich während des Zuführens des Sterilisationsmediums an der mindestens einen stationären Düse vorbeibewegt.

8. Verfahren nach Anspruch 6 oder Anspruch 7, wobei:
die mindestens eine Düse (30) mindestens eine bewegbare Düse aufweist und die mindestens eine bewegbare Düse sich während des Zuführens des Sterilisationsmediums mit der Dose (12) mitbewegt.

9. Verfahren nach einem der Ansprüche 6 bis 8, wobei:
eine Sterilisationsmedium-Austrittsmenge an der mindestens einen Düse (30) konstant ist, oder
eine Sterilisationsmedium-Austrittsmenge an der mindestens einen Düse (30) in Abhängigkeit von einer Transportgeschwindigkeit der Dose (12) entlang der mindestens einen Düse (30) angepasst wird.

10. Verfahren nach einem der Ansprüche 6 bis 9, wobei:
das Sterilisationsmedium von der mindestens einen Düse (30) gepulst abgegeben wird, vorzugsweise mit:
- einer Pulsfrequenz, die an eine Frequenz von sich an der mindestens einen Düse (30) vorbeibewegenden Dosen (12) angepasst ist; und/oder
- mit einer Pulsdauer, die an eine Transportgeschwindigkeit, mit der die Dose an der mindestens einen Düse (30) vorbeibewegt wird, angepasst ist; und/oder
- mit einem oder mehreren Pulsen je Dose (12).

11. Verfahren nach einem der Ansprüche 6 bis 10, wobei die mindestens eine Düse (30) aufweist:
eine Düse, die das Sterilisationsmedium senkrecht von oben und mittig in die Dose (12) zuführt; und/oder
eine Düse, die das Sterilisationsmedium senkrecht von oben und außermittig in die Dose (12) zuführt; und/oder
eine Düse, die das Sterilisationsmedium schräg angewinkelt zu einer Hochachse der Dose (12) von oben in die Dose (12) zuführt, bevorzugt angewinkelt zwischen 10° und 20°, besonders bevorzugt angewinkelt um 15°.

12. Verfahren nach einem der vorherigen Ansprüche, ferner aufweisend:
Sterilisieren mindestens eines Abschnitts der Füllstation (24) mittels des aus der Dose (12) ausgespülten Sterilisationsmediums, wobei vorzugsweise der mindestens eine Abschnitt einen Füllgutauslass der Füllstation (24) und/oder eine Dichtfläche der Füllstation (24) aufweist.

13. Verfahren nach einem der vorherigen Ansprüche, wobei:
die gefüllte Dose (12) in der Dosenbehandlungsanlage (10) nicht pasteurisiert wird.

14. Dosenbehandlungsanlage (10), vorzugsweise ausgebildet zum Ausführen eines Verfahrens nach einem der Ansprüche 1 bis 13, wobei die Dosenbehandlungsanlage (10) aufweist:
eine Sterilisationsvorrichtung (14), die zum Zuführen eines, vorzugsweise gasförmigen, Sterilisationsmediums, vorzugsweise aufweisend Wasserstoffperoxid, in Dosen (12) ausgebildet ist; und
eine Dosenfüllvorrichtung (22), die stromabwärts von der Sterilisationsvorrichtung (14) angeordnet ist und mindestens eine Füllstation (24), die zum Spülen der Dosen (12) zum Ausspülen des Sterilisationsmediums aus den Dosen (12) und zum Füllen der Dosen (12) mit einem Füllgut ausgebildet ist, aufweist.

15. Dosenbehandlungsanlage (10) nach Anspruch 14, wobei:
die Dosenbehandlungsanlage (10) ferner einen Dosenförderer (16) aufweist, der die Sterilisationsvorrichtung (14) direkt mit der Dosenfüllvorrichtung (22) verbindet; und/oder
die Dosenbehandlungsanlage (10) frei von einer Pasteurisiervorrichtung zum Pasteurisieren der Dosen (12) nach dem Füllen ist; und/oder
die Dosenbehandlungsanlage (10) vom Beginn der Sterilisationsvorrichtung (14) bis hin zur Dosenfüllvorrichtung (22) keine Vorrichtung aufweist, die zum Entfernen des Sterilisationsmediums aus den Dosen (12) ausgebildet ist.
